# NEUE EUROPÄISCHE PATENTSCHRIFT

(11) **EP 3 080 308 B2**
(45) Veröffentlichungstag und Bekanntmachung des Hinweises auf die Entscheidung über den Einspruch: **16.07.2025**
(45) Hinweis auf die Patenterteilung: 06.05.2020
(21) Anmeldenummer: 14815581.5
(22) Anmeldetag: 11.12.2014
(51) Int. Cl.: C21C 5/38, C21B 5/06, C21B 7/00, C21C 5/28, C21C 5/40, C21C 1/00, C25B 15/08, C25B 1/04

(54) **ANLAGENVERBUND ZUR STAHLERZEUGUNG UND VERFAHREN ZUM BETREIBEN DES ANLAGENVERBUNDES**
COMBINED SYSTEM FOR PRODUCING STEEL AND METHOD FOR OPERATING THE COMBINED SYSTEM
RÉSEAU D'INSTALLATIONS POUR LA PRODUCTION D'ACIER ET PROCÉDÉ POUR FAIRE FONCTIONNER CE RÉSEAU D'INSTALLATIONS

(30) Priorität: 12.12.2013 DE 102013113921
(43) Veröffentlichungstag der Anmeldung: 19.10.2016
(73) Patentinhaber: thyssenkrupp AG, 45143 Essen (DE)
(72) Erfinder: ACHATZ, Reinhold, 45259 Essen (DE); WAGNER, Jens, 60439 Frankfurt a.M. (DE); OLES, Markus, 45527 Hattingen (DE); SCHMÖLE, Peter, 44141 Dortmund (DE); KLEINSCHMIDT, Ralph, 45472 Mülheim a.d. Ruhr (DE); KRÜGER, Matthias Patrick, 44625 Herne (DE); KROTOV, Denis, 44263 Dortmund (DE); VON MORSTEIN, Olaf, 45149 Essen (DE)
(74) Vertreter: Andrejewski - Honke Patent- und Rechtsanwälte Partnerschaft mbB
(86) Internationale Anmeldenummer: PCT/EP2014/003319
(87) Internationale Veröffentlichungsnummer: WO 2015/086153

(56) Entgegenhaltungen:
- EP-A1- 2 543 743
- EP-A1- 2 657 215
- EP-A1- 2 781 584
- EP-A2- 0 200 880
- WO-A1-00/05421
- WO-A1-2009/058028
- WO-A1-2011/018124
- WO-A1-2013/037444
- DE-A1- 102013 004 996
- JP-A- 2011 225 969
- US-A1- 2006 027 043
- HAMID GHANBARI ET AL: "Optimal design and operation of a steel plant integrated with a polygeneration system", AI CH E JOURNAL, vol. 59, no. 10, 1 October 2013 (2013-10-01), US, pages 3659 - 3670, XP055501257, ISSN: 0001-1541, DOI: 10.1002/aic.14098
- HAMID GHANBARI ET AL: "Optimal design and operation of a steel plant integrated with a polygeneration system", AI CH E JOURNAL, vol. 59, no. 10, 1 October 2013 (2013-10-01), US, pages 3659 - 3670, XP055501257, ISSN: 0001-1541, DOI: 10.1002/aic.14098
- Power.Feb 23, 2012, Turbine Suppliers Pursue a Different Niche: Steel Mills
- SCHMOELE P ET AL: "ECOLOGICAL HOT METAL PRODUCTION USING COKE PLANT AND BLAST FURNACE ROUTE//PRODUCTION ECOLOGIQUE DE FONTE PAR LA FILIERE COKERIE ET HAUT-FOURNEAU", REVUE DE METALLURGIE - CAHIERS D'INFORMATIONS TECHNIQUES, REVUE DE METALLURGIE. PARIS, FR, vol. 102, no. 3, 1 March 2005 (2005-03-01), pages 171 - 182, XP001230940, ISSN: 0035-1563, DOI: 10.1051/METAL:2005140

## Beschreibung

Die Erfindung betrifft einen Anlagenverbund zur Stahlerzeugung sowie ein Verfahren zum Betreiben des Anlagenverbundes US 2006/027043 A1, WO 00/05421 A1 und HAMID GHANBARI ET AL: "Optimal design and operation of a steel plant integrated with a polygeneration system", offenbaren integrierte Anlagenverbände zur Stahlerzeugung, wobei Gase aus der Stahlerzeugung in nachgeschaltete Chemieanlagen geleitet werden.

Der Anlagenverbund zur Stahlerzeugung umfasst zumindest einen Hochofen zur Roheisenerzeugung, ein Konverterstahlwerk zur Rohstahlerzeugung, und ein Gasleitungssystem für Gase, die bei der Roheisenerzeugung und/oder der Rohstahlerzeugung anfallen. Der Anlagenverbund kann ferner ein Kraftwerk zur Stromerzeugung aufweisen, welches als Gasturbinenkraftwerk oder Gasturbinen- und Dampfturbinenkraftwerk ausgelegt ist und mit einem Gas betrieben wird, das zumindest eine Teilmenge des bei der Roheisenerzeugung im Hochofen anfallenden Hochofengichtgases und/oder eine Teilmenge des in dem Konverterstahlwerk anfallenden Konvertergases umfasst.

Im Hochofen wird aus Eisenerzen, Zuschlägen sowie Koks und anderen Reduktionsmitteln wie Kohle, Öl, Gas, Biomassen aufbereiteten Altkunststoffen oder sonstigen Kohlenstoff und/oder Wasserstoff enthaltenen StoffenRoheisen gewonnen. Als Produkte der Reduktionsreaktionen entstehen zwangsläufig CO, CO₂, Wasserstoff und Wasserdampf. Ein aus dem Hochofenprozess abgezogenes Hochofengichtgas weist neben den vorgenannten Bestandteile häufig einen hohen Gehalt an Stickstoff auf. Die Gasmenge und die Zusammensetzung des Hochofengichtgases ist abhängig von den Einsatzstoffen und der Betriebsweise und unterliegt Schwankungen. Typischerweise enthält Hochofengichtgas jedoch 35 bis 60 Vol.-% N₂, 20 bis 30 Vol.-% CO, 20 bis 30 Vol.-% CO₂ und 2 bis 15 Vol.-% H₂. Rund 30 bis 40% des bei der Roheisenerzeugung entstehenden Hochofengichtgases wird im Regelfall zum Aufheizen des Heißwindes für den Hochofenprozess in Winderhitzern eingesetzt; die verbleibende Gichtgasmenge kann in anderen Werksbereichen extern zu Heizzwecken oder zur Stromerzeugung genutzt werden.

Im Konverterstahlwerk, das dem Hochofenprozess nachgeschaltet ist, wird Roheisen zu Rohstahl umgewandelt. Durch Aufblasen von Sauerstoff auf flüssiges Roheisen werden störende Verunreinigungen wie Kohlenstoff, Silizium, Schwefel und Phosphor entfernt. Da die Oxidationsprozesse eine starke Wärmeentwicklung verursachen, wird häufig Schrott in Mengen bis zu 25% bezogen auf das Roheisen als Kühlmittel zugesetzt. Ferner werden Kalk zur Schlackenbildung und Legierungsmittel zugegeben. Aus dem Stahlkonverter wird ein Konvertergas abgezogen, welches einen hohen Gehalt an CO aufweist und ferner Stickstoff, Wsserstoff und CO₂ enthält. Eine typische Konvertergaszusammensetzung weist 50 bis 70 Vol.-% CO, 10 bis 20 Vol.-% N₂, ca. 15 Vol.-% CO₂ und ca. 2 Vol.-% H₂ auf. Das Konvertergas wird entweder abgefackelt oder bei modernen Stahlwerken aufgefangen und einer energetischen Nutzung zugeführt.

Der Anlagenverbund kann optional im Verbund mit einer Kokerei betrieben werden. In diesem Fall umfasst der eingangs beschriebene Anlagenverbund zusätzlich eine Koksofenanlage, in der Kohle durch einen Verkokungsprozess in Koks umgewandelt wird. Bei der Verkokung von Kohle zu Koks fällt ein Koksofengas an, welches einen hohen Wasserstoffgehalt und beachtliche Mengen an CH₄ enthält. Typischerweise enthält Koksofengas 55 bis 70 Vol.-% H₂, 20 bis 30 Vol.-% CH₄, 5 bis 10 Vol.-% N₂ und 5 bis 10 Vol.-% CO. Zusätzlich weist das Koksofengas Anteile von CO₂, NH₃ und H₂S auf. In der Praxis wird das Koksofengas in verschiedenen Werksbereichen zu Heizzwecken und im Kraftwerksprozess zur Stromerzeugung genutzt. Darüber hinaus ist es bekannt, Koksofengas zusammen mit Hochofengichtgas oder mit Konvertergas zur Erzeugung von Synthesegasen zu verwenden. Gemäß einem aus WO 2010/136313 A1 bekannten Verfahren wird Koksofengas aufgetrennt in einen wasserstoffreichen Gasstrom und einen CH₄ und CO enthaltenen Restgasstrom, wobei der Restgasstrom dem Hochofenprozess zugeführt wird und der wasserstoffreiche Gasstrom mit Hochofengichtgas gemischt und zu einem Synthesegas weiterverarbeitet wird. Aus EP 0 200 880 A2 ist es bekannt, Konvertergas und Koksofengas zu mischen und als Synthesegas für eine Methanolsynthese zu nutzen.

In einem integriertem Hüttenwerk, welches im Verbund mit einer Kokerei betrieben wird, werden etwa 40 bis 50% der als Hochofengichtgas, Konvertergas und Koksofengas anfallenden Rohgase für verfahrenstechnische Prozesse eingesetzt. Etwa 50 bis 60% der entstehenden Gase werden dem Kraftwerk zugeführt und zur Stromerzeugung genutzt. Der im Kraftwerk erzeugte Strom deckt den Strombedarf für die Roheisen- und Rohstahlerzeugung. Im Idealfall ist die Energiebilanz geschlossen, so dass abgesehen von Eisenerzen und Kohlenstoff in Form von Kohle und Koks als Energieträger kein weiterer Eintrag von Energie notwendig ist und außer Rohstahl und Schlacke kein Produkt den Anlagenverbund verlässt.

Vor diesem Hintergrund liegt der Erfindung die Aufgabe zugrunde, die Wirtschaftlichkeit des Gesamtprozesses weiter zu verbessern und einen Anlagenverbund anzugeben, mit dem es möglich ist, die Kosten für die Stahlerzeugung zu reduzieren.

Ausgehend von einem Anlagenverbund zur Stahlerzeugung mit einem Hochofen zur Roheisenerzeugung, einem Konverterstahlwerk zur Rohstahlerzeugung und einem Gasleitungssystem für Gase, die bei der Roheisenerzeugung und/oder der Rohstahlerzeugung anfallen, ist erfindungsgemäß eine an das Gasleitungssystem angeschlossene Chemie- oder Biotechnologieanlage sowie eine Anlage zur Wasserstofferzeugung vorgesehen, wobei die Anlage zur Wasserstofferzeugung durch eine wasserstoffführende Leitung mit dem Gasleitungssystem verbunden ist. Vorteilhafte Ausgestaltungen des erfindungsgemäßen Anlagenverbundes werden in den Patentansprüchen 2 bis 4 beschrieben.

Gegenstand der Erfindung ist auch ein Verfahren nach Anspruch 5 zum Betreiben eines Anlagenverbundes zur Stahlerzeugung, der zumindest einen Hochofen zur Roheisenerzeugung, ein Konverterstahlwerk, eine Chemieanlage oder Biotechnologieanlage sowie eine Anlage zur Wasserstofferzeugung aufweist. Gemäß dem erfindungsgemäßen Verfahren wird zumindest eine Teilmenge eines bei der Roheisenerzeugung im Hochofen anfallenden Hochofengichtgases und/oder eine Teilmenge eines bei der Rohstahlerzeugung anfallenden Konvertergases nach einer Gaskonditionierung als Nutzgas zur Herstellung chemischer Produkte in einer Chemieanlage oder Biotechnologieanlage verwendet. Dabei wird das Nutzgas vor seiner Verwendung als Synthesegas mit Wasserstoff angereichert, der in der Anlage zur Wasserstofferzeugung gebildet wird. Aus Konvertergas oder Hochofengichtgas oder einem Mischgas, welches aus Hochofengichtgas und Konvertergas gebildet wird, können im Wesentlichen aus CO und H2 bestehende Synthesegase erzeugt werden, deren Zusammensetzung auf einen nachfolgenden Prozess in der Chemieanlage oder der Biotechnologieanlage abgestimmt ist. Mit einer gezielten Zugabe von Wasserstoff, der innerhalb des Anlagenverbundes erzeugt wird, kann das Verhältnis aus CO und Wasserstoff sehr genau eingestellt werden und über einen großen Parameterbereich variiert werden.

In der Chemieanlage können chemische Produkte aus Synthesegasen erzeugt werden, welche jeweils die Komponenten des Endproduktes enthalten. Chemische Produkte können beispielsweise Ammoniak oder Methanol oder auch andere Kohlenwasserstoffverbindungen sein.

Zur Herstellung von Ammoniak muss ein Synthesegas bereitgestellt werden, welches Stickstoff und Wasserstoff im richtigen Verhältnis enthält. Der Stickstoff kann aus Hochofengichtgas gewonnen werden. Als Wasserstoffquelle kann Hochofengichtgas oder Konvertergas verwendet werden, wobei Wasserstoff durch Konvertierung des CO-Anteils durch eine Wasser-Gas-Shift-Reaktion (CO + H₂O ⇄ CO₂ + H₂) erzeugt wird. Zur Herstellung von Kohlenwasserstoffverbindungen, beispielsweise Methanol, muss ein im Wesentlichen aus CO und/oder CO₂ und H₂ bestehendes Synthesegas bereitgestellt werden, welches die Komponenten Kohlenmonoxid und/oder Kohlendioxid und Wasserstoff im richtigen Verhältnis enthält. Das Verhältnis wird häufig durch den Modul (H₂ - CO₂) / (CO + CO₂) beschrieben. Der Wasserstoff kann beispielsweise durch Konvertierung des CO-Anteils im Hochofengichtgas durch eine Wasser-Gas-Shift-Reaktion erzeugt werden. Zur Bereitstellung von CO kann Konvertergas herangezogen werden. Als CO₂-Quelle kann Hochofengichtgas und/oder Konvertergas dienen.

Bei den vorstehend beschriebenen Konzepten kann allerdings der C-Gehalt bzw. N-Gehalt des Mischgases nicht vollständig genutzt werden, da ein Wasserstoff-Unterschuß vorliegt. Um den C-Gehalt bzw. N-Gehalt der bei der Roheisenerzeugung und/oder der Rohstahlerzeugung anfallenden Gase vollständig für die Herstellung von chemischen Produkten nutzen zu können, wird erfindungsgemäß Wasserstoff zudosiert, der in einer Anlage zur Wasserstofferzeugung gebildet wird. Die Wasserstofferzeugung erfolgt durch Wasserelektrolyse, wobei die Wasserelektrolyse zweckmäßig mit elektrischem Strom betrieben wird, der aus erneuerbarer Energie erzeugt wurde. Bei der Wasserelektrolyse entsteht auch Sauerstoff, der im Hochofen zur Roheisenerzeugung und/oder im Konverterstahlwerk zur Rohstahlerzeugung genutzt werden kann.

Im Rahmen der Erfindung liegt es auch, dass Synthesegas aus Konvertergas erzeugt und mit Wasserstoff angereichert wird. Durch die Anreicherung mit Wasserstoff, der innerhalb des Anlagenverbundes entsprechend dem Wasserstoffbedarf erzeugt wird, kann der H₂-Gehalt des Konvertergases auf jeden gewünschten Wert eingestellt werden.

Ferner besteht die Möglichkeit, aus Hochofengichtgas und Konvertergas ein Mischgas herzustellen, welches nach einer Gaskonditionierung und Anreicherung mit Wasserstoff als Synthesegas zur Herstellung chemischer Produkte verwendet wird. Dabei ist es zweckmäßig, den Wasserstoff durch Wasserelektrolyse unter Verwendung von Strom aus erneuerbarer Energie zu erzeugen.

Anstelle einer Chemieanlage zur Erzeugung von Produkten aus Synthesegas kann im Rahmen der Erfindung auch eine biotechnologische Anlage eingesetzt werden. Hierbei handelt es sich um eine Anlage zur Fermentation von Synthesegas. Das Synthesegas wird über eine Fermentation biochemisch genutzt, wobei Produkte wie Alkohole (Ethanol, Butanol), Aceton oder organische Säuren hergestellt werden können. Auch diese Produkte, die durch Fermentation von Synthesegas erzeugt werden, sind im vorliegenden Fall nur beispielhaft genannt.

Gemäß einer bevorzugten Ausführung der Erfindung umfasst der Anlagenverbund zusätzlich eine Koksofenanlage. Wenn die Roheisenerzeugung und die Rohstahlerzeugung im Verbund mit einer Kokerei betrieben wird, kann eine Teilmenge des bei der Roheisenerzeugung anfallenden Hochofengichtgases und/oder eine Teilmenge des im Konverterstahlwerk angefallenen Konvertergases mit einer Teilmenge des in der Koksofenanlage entstehenden Koksofengases gemischt werden und das Mischgas als Nutzgas verwendet werden. Zur Erzeugung eines Synthesegases beispielsweise für die Ammoniaksynthese kann als Nutzgas eine Mischung aus Koksofengas und Hochofengichtgas oder ein Mischgas aus Koksofengas, Konvertergas und Hochofengichtgas verwendet werden. Zur Herstellung von Kohlenwasserstoffverbindungen eignet sich ein Mischgas aus Koksofengas und Konvertergas oder ein Mischgas aus Koksofengas, Konvertergas und Hochofengichtgas. Dabei sind die beschriebenen Chemieprodukte, die in einer Chemieanlage aus Hochofengichtgas, Konvertergas und Koksofengas hergestellt werden können, nur Anwendungsbeispiele zur Erläuterung der in den Patentansprüchen beschriebenen Verfahrensvarianten.

Die Rohgase - Koksofengas, Konvertergas und/oder Hochofengichtgas - können einzeln oder in Kombinationen als Mischgas aufbereitet und dann als Synthesegas der Chemieanlage zugeführt werden. Die Aufbereitung insbesondere von Kokosofengas umfasst eine Gasreinigung zur Abtrennung störender Inhaltsstoffe, insbesondere Teer, Schwefel und Schwefelverbindungen, aromatischen Kohlenwasserstoffen (BTX) und hochsiedenden Kohlenwasserstoffen. Zum Herstellen des Synthesegases ist ferner eine Gaskonditionierung notwendig. Im Rahmen der Gaskonditionierung wird der Anteil der Komponenten CO, CO₂, H₂ innerhalb des Rohgases verändert. Die Gaskonditionierung umfasst beispielsweise eine Druckwechseladsorption zur Abtrennung und Anreicherung von H₂ und/oder eine Wasser-Gas-Shift-Reaktion zur Umwandlung von CO in Wasserstoff und/oder einen Steam-Reformer zur Umwandlung des CH₄-Anteils in CO und Wasserstoff im Koksofengas.

Gemäß einer bevorzugten Ausführung der Erfindung umfasst der Anlagenverbund ein Kraftwerk zur Stromerzeugung, das als Gasturbinenkraftwerk oder Gasturbinen-/Dampfturbinenkraftwerk ausgelegt ist und mit einem Gas betrieben wird, welches zumindest eine Teilmenge des bei der Roheisenerzeugung im Hochofen anfallenden Hochofengichtgases und/oder einer Teilmenge des im Konverterstahlwerk anfallenden Konvertergases umfasst. Das Kraftwerk zur Stromerzeugung und die Chemieanlage oder Biotechnologieanlage sind bezogen auf die Strömungsführung der Gase parallel geschaltet. Die einerseits dem Kraftwerk und andererseits der Chemie- oder Biotechnologieanlage zugeführten Gasströme sind regelbar.

Bei dem erfindungsgemäßen Verfahren wird zumindest eine Teilmenge des bei der Roheisenerzeugung im Hochofen anfallenden Hochofengichtgases und/ oder eine Teilmenge des in dem Konverterstahlwerk anfallenden Konvertergases als Rohgas genutzt, um daraus durch chemische Reaktionen in einer Chemieanlage oder durch biochemische Prozesse in einer biotechnologischen Anlage Produkte, das heißt Wertstoffe, zu erzeugen. Als Konsequenz aus der Nutzung eines Teils dieser Gase fehlt dem Anlagenverbund Strom, der extern bezogen werden muss. Der extern bezogene Strom kann aus konventionellen Kraftwerken stammen oder aus erneuerbaren Energien gewonnen werden. Vorzugsweise wird der extern bezogene Strom vollständig oder zumindest teilweise aus erneuerbarer Energie gewonnen und stammt beispielsweise aus Windkraftanlagen, Solaranlagen, geothermischen Kraftwerken, Wasserkraftwerken, Gezeitenkraftwerken und dergleichen. Zur Erreichung eines möglichst wirtschaftlichen Betriebs des Anlagenverbunds wird Strom in Zeiten niedriger Strompreise zugekauft und zur Versorgung des Anlagenverbunds verwendet und wird der nicht zur Stromerzeugung genutzte Teil des Nutzgases nach einer Gaskonditionierung in der Chemieanlage oder der biotechnologischen Anlage zur Herstellung von chemischen Produkten verwendet. In Zeiten hoher Strompreise wird das Nutzgas hingegen dem Kraftwerk vollständig oder zumindest zu einem großen Teil zugeführt, um Strom zur Versorgung des Anlagenverbundes zu erzeugen. Die Chemieanlage oder biotechnologische Anlage wird in Zeiten hoher Strompreise entsprechend heruntergefahren. Entsprechendes gilt für die mit elektrischem Strom betriebene Wasserelektrolyse. Wenn bei hohen Strompreisen die Chemieanlage heruntergefahren wird, ist auch der Bedarf an Wasserstoff gering. Wenn die Chemieanlage dagegen bei niedrigen Strompreisen mit einer großen Produktionsleistung betrieben wird, kann auch der Wasserstoff durch Wasserelektrolyse kostengünstig erzeugt werden. Zum Betrieb des Verfahrens wird eine Regelung vorgesehen, die den wechselseitigen Betrieb des Kraftwerkes einerseits und der Chemieanlage oder biotechnologischen Anlage andererseits in Abhängigkeit einer variablen Prozessgröße festgelegt. Die Prozessgröße wird vorzugsweise in Abhängigkeit einer Funktion bestimmt, welche den Preis für den extern bezogenen Strom und die Kosten für die Erzeugung des Kraftwerkstroms als Variablen enthält.

Das erfindungsgemäße Verfahren ermöglicht einen wirtschaftlichen Betrieb des Anlagenverbundes. Dabei nutzt das erfindungsgemäße Verfahren insbesondere auch aus, dass der Wirkungsgrad eines Kraftwerksprozess zur Erzeugung von Strom schlechter ist als der Wirkungsgrad einer Chemieanlage oder einer biotechnologischen Anlage, in der durch chemische Reaktionen oder durch biochemische Prozesse aus Synthesegas Chemieprodukte hergestellt werden.

Die Leistung der Chemieanlage oder der biotechnologischen Anlage wird in Abhängigkeit der dieser Anlage zugeführten Synthesegasmenge geregelt. Eine wesentliche Herausforderung für die Chemieanlage ist die dynamische Fahrweise bei wechselnden Anlagenlasten. Die Betriebsweise bei wechselnden Anlagenlasten kann insbesondere dadurch realisiert werden, dass die Chemieanlage eine Mehrzahl parallel geschalteter kleiner Einheiten aufweist, die je nach zur Verfügung stehendem Nutzgasmengenstrom einzeln zu- oder abgeschaltet werden.

Die Verwendung einer biotechnologischen Anlage hat den Vorteil, dass eine biotechnologische Anlage flexibler hinsichtlich Lastwechsel ist als eine Chemieanlage.

Unter die Erfindung fällt ferner die Verwendung einer Chemie- oder Biotechnologieanlage zur Ankopplung an ein Hüttenwerk nach Anspruch **14**.

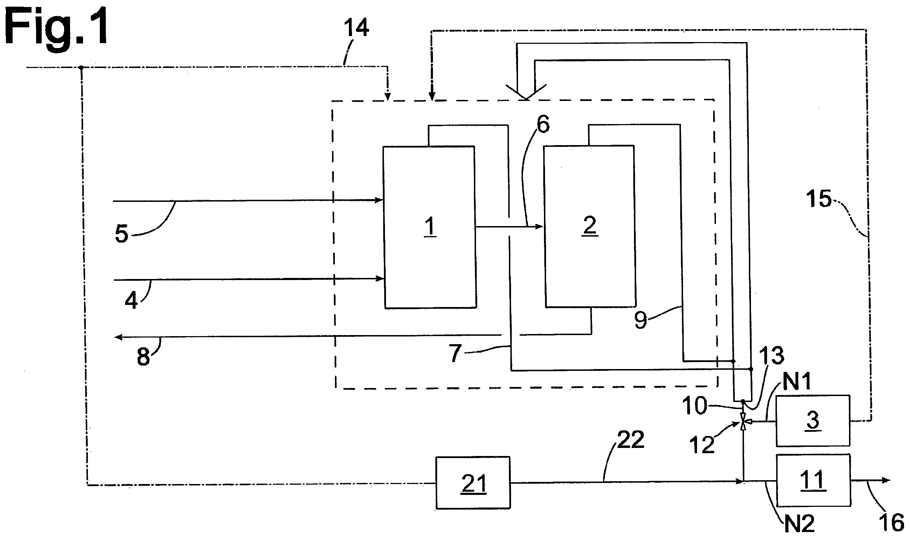

Im Folgenden wird die Erfindung anhand einer lediglich ein Ausführungsbeispiel darstellenden Zeichnung erläutert. Es zeigt schematisch
- **Fig. 1**: ein stark vereinfachtes Blockschaltbild eines Anlagenverbundes zur Stahlerzeugung mit einem Hochofen zur Roheisenerzeugung, einem

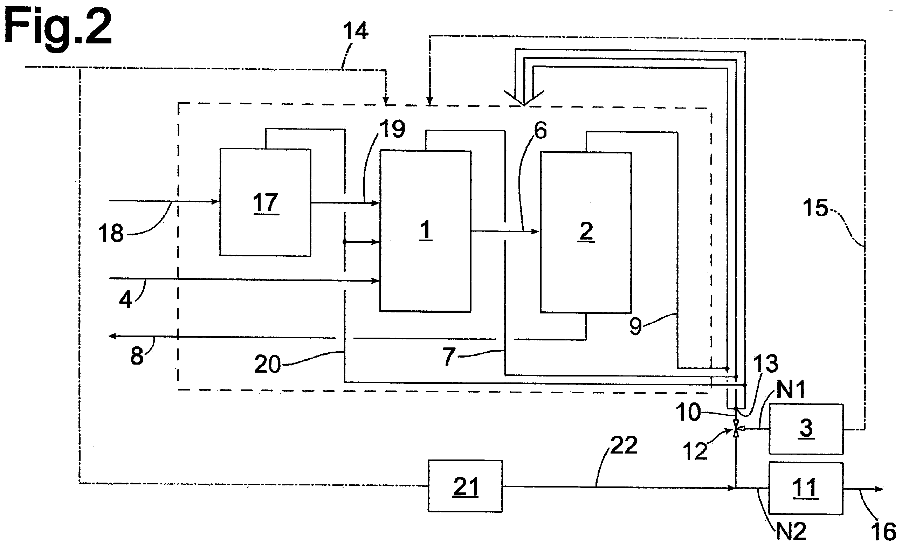

- **Fig. 2**: Konverterstahlwerk zur Rohstahlerzeugung, einem Kraftwerk, einer Chemie- oder Biotechnologieanlage und einer Anlage zur Erzeugung von Wasserstoff, das stark vereinfachte Blockschaltbild eines Anlagenverbundes, der zusätzlich zu einem Hochofen zur Roheisenerzeugung, einem Konverterstahlwerk zur Rohstahlerzeugung, einem Kraftwerk, einer Chemie- oder Biotechnologieanlage und einer Anlage zur Erzeugung von Wasserstoff auch eine Koksofenanlage umfasst.

Der in Fig. 1 dargestellte Anlagenverbund zur Stahlerzeugung umfasst einen Hochofen 1 zur Roheisenerzeugung, ein Konverterstahlwerk 2 zur Rohstahlerzeugung, ein Kraftwerk 3 zur Stromerzeugung und eine Chemie- oder Biotechnologieanlage 11.

Im Hochofen 1 wird im Wesentlichen aus Eisenerz 4 und Reduktionsmitteln 5, insbesondere Koks und Kohle, Roheisen 6 gewonnen. Durch Reduktionsreaktionen entsteht ein Hochofengichtgas 7, welches als Hauptbestandteile Stickstoff, CO, CO₂ und H₂ enthält. Im Konverterstahlwerk 2, das dem Hochofenprozess nachgeschaltet ist, wird Roheisen 6 zu Rohstahl 8 umgewandelt. Durch Aufblasen von Sauerstoff auf das flüssige Roheisen werden störende Verunreinigungen, insbesondere Kohlenstoff, Silizium und Phosphor entfernt. Zur Kühlung kann Schrott in Mengen bis zu 25% bezogen auf die Roheisenmenge zugeführt werden. Ferner werden Kalk zur Schlackenbildung und Legierungsmittel zugegeben. Am Kopf des Konverters wird ein Konvertergas 9 abgezogen, welches einen sehr hohen Anteil an CO aufweist.

Das Kraftwerk 3 ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen 1 anfallenden Hochofengichtgases 7 und/oder eine Teilmenge des im Konverterstahlwerk 2 anfallenden Konvertergases 9 umfasst. Zur Führung der Gase ist ein Gasleitungssystem vorgesehen.

Gemäß einer in Fig. 1 dargestellten Gesamtbilanz wird dem Anlagenverbund Kohlenstoff als Reduktionsmittel 5 in Form von Kohle und Koks sowie Eisenerz 4 zugeführt. Als Produkte fallen Rohstahl 8 und Rohgase 7, 9 an, die sich in Menge, Zusammensetzung, Heizwert und Reinheit unterscheiden und an verschiedenen Stellen im Anlagenverbund wieder eingesetzt werden. Bei einer Gesamtbetrachtung wird 40 bis 50%, zumeist etwa 45%, der Rohgase 7, 9 wieder in den Hüttenprozess zur Roheisenerzeugung oder Rohstahlerzeugung zurückgeführt. Zwischen 50 und 60%, zumeist etwa 55%, der Rohgase 7, 9 kann zum Betrieb des Kraftwerkes 3 genutzt werden. Das mit einem Mischgas 10 aus Hochofengichtgas 7 und Konvertergas 9 betriebene Kraftwerk 3 wird so ausgelegt, dass es den Strombedarf des Anlagenverbundes decken kann.

Gemäß der Darstellung in Fig. 1 ist eine Chemie- oder Biotechnologieanlage 11 vorgesehen, die an das Gasleitungssystem angeschlossen ist und hinsichtlich der Gasversorgung parallel zu dem Kraftwerk 3 geschaltet ist. Das Gasleitungssystem weist eine betrieblich steuerbare Gasweiche 12 zur Aufteilung der dem Kraftwerk 3 und der Chemie- oder Biotechnologieanlage 11 zugeführten Gasmengeströme auf. In Strömungsrichtung vor der Gasweiche ist eine Mischvorrichtung 13 zur Herstellung des aus Hochofengichtgas 7 und Konvertergas 9 bestehenden Mischgases 10 vorgesehen.

Bei dem in Fig. 1 dargestellten Anlagenverbund wird zumindest eine Teilmenge des bei der Roheisenerzeugung im Hochofen 1 anfallenden Hochofengichtgases 7 und eine Teilmenge des bei der Rohstahlerzeugung anfallenden Konvertergases 9 als Nutzgas zum Betrieb des Kraftwerkes 3 und der Chemie- oder Biotechnologieanlage 11 verwendet. Zur Deckung des Strombedarfes des Anlageverbundes wird extern bezogener Strom 14 und Kraftwerkstrom 15, der von dem Kraftwerk 3 des Anlagenverbundes erzeugt wird, herangezogen. Der Stromanteil des extern bezogenen Stroms 14 bezogen auf den gesamten Strombedarf des Anlagenverbundes wird als variable Prozessgröße festgelegt und die dem Kraftwerk 3 zugeführte Nutzgasmenge N1 wird in Abhängigkeit dieser Prozessgröße bestimmt. Der nicht zur Stromerzeugung genutzte Teil des Nutzgases N2 wird nach einer Gaskonditionierung als Synthesegas zur Herstellung chemischer Produkte 16 verwendet oder nach einer Gaskonditionierung der biotechnologischen Anlage zugeführt und für biochemische Prozesse genutzt.

Der extern bezogene Strom 14 wird vorzugsweise vollständig oder zumindest teilweise aus erneuerbarer Energie gewonnen und stammt beispielsweise aus Windkraftanlagen, Solaranlagen, Wasserkraftwerken und dergleichen. Die Prozessgröße, auf deren Grundlage die dem Kraftwerksprozess zugeführte Nutzgasmenge N1 festgelegt wird, wird in Abhängigkeit einer Funktion bestimmt, welche den Preis für den extern bezogenen Strom und die Kosten für die Erzeugung des Kraftwerkstromes 15 als Variablen enthält. Zur Erreichung eines möglichst wirtschaftlichen Betriebs des Anlagenverbundes wird elektrischer Strom in Zeiten niedriger Strompreise als externer Strom 14 zugekauft und zur Stromversorgung des Anlagenverbundes verwendet, wobei der nicht zur Stromerzeugung genutzte Teil des Nutzgases N2 der Chemie- oder Biotechnologieanlage 11 zugeführt und nach einer Gaskonditionierung als Synthesegas zur Herstellung chemischer Produkte 16 verwendet wird. In Zeiten hoher Strompreise werden die bei der Roheisenerzeugung und Rohstahlerzeugung anfallenden Rohgase 7, 9 dem Kraftwerk 3 zugeführt, um den Strom zur Versorgung des Anlagenverbundes zu erzeugen. Die Chemieanlage 11 oder die alternativ vorgesehene biotechnologische Anlage wird in Zeiten hoher Strompreise entsprechend heruntergefahren.

Damit der Kohlenstoffgehalt und der Stickstoffgehalt der beim Betrieb des Anlagenverbundes anfallenden Rohgase vollständig zur Herstellung von chemischen Produkten genutzt werden kann, muss Wasserstoff zum Ausgleich eines Wasserstoffunterschusses zugeführt werden. Der Anlagenverbund weist daher zusätzlich eine Anlage 21 zur Wasserstofferzeugung auf, die durch eine wasserstoffführende Leitung 22 mit dem Gasleitungssystem verbunden ist. Die Anlage 21 zur Wasserstofferzeugung **ist erfindungsgemäß** eine Elektrolyseanlage zur Wasserelektrolyse. Der Betrieb einer Wasserelektrolyse ist energieintensiv und wird daher primär in Zeiten niedriger Strompreise in Betrieb genommen, zu denen auch die Chemieanlage 11 oder Biotechnologieanlage betrieben wird und das Kraftwerk 3 heruntergefahren ist. Der zusätzlich erzeugte Wasserstoff wird der Chemieanlage 11 zusammen mit dem Nutzgas als Synthesegas zugeführt. Dadurch kann die Kapazität der Chemieanlage 11 deutlich gesteigert werden. Entsprechendes gilt, wenn anstelle der Chemieanlage 11 eine biotechnologische Anlage vorgesehen wird.

In dem Ausführungsbeispiel der Fig. 2 umfasst der Anlagenverbund zusätzlich eine Koksofenanlage 17. Bei der Verkokung von Kohle 18 zu Koks 19 fällt Koksofengas 20 an, welches einen hohen Anteil an Wasserstoff und CH₄ enthält. Teile des Koksofengases 20 können für die Beheizung der Winderhitzer im Hochofen 1 genutzt werden. Das Gasleitungsystem schließt eine Gasverteilung für das Koksofengas 20 ein. In Strömungsrichtung vor der Gasweiche 12 ist eine Mischvorrichtung 13 zur Herstellung eines aus Hochofengichtgas 7, Konvertergas 9 und Koksofengas 20 bestehenden Mischgases 10 vorgesehen. Mit der Gasweiche 12 sind die dem Kraftwerk 3 und der Chemie- oder Biotechnologieanlage 11 zugeführten Gasmengenströme steuerbar.

Beim Betrieb der in Fig. 2 dargestellten Anlage wird eine Teilmenge des bei der Roheisenerzeugung anfallenden Hochofengichtgases 7 und/oder eine Teilmenge des im Konverterstahlwerk anfallenden Konvertergases 9 mit einer Teilmenge des in der Koksofenanlage 17 entstehenden Koksofengases 20 gemischt. Das Mischgas 10 wird zum Betrieb des Kraftwerkes 3 und nach einer Gaskonditionierung und Anreicherung mit Wasserstoff als Synthesegas in der Chemieanlage 11 oder biotechnologischen Anlage verwendet.

Das Hochofengichtgas 7, das Konvertergas 9 und das Koksofengas 20 können beliebig miteinander kombiniert werden. Die Kombination der Gasströme 7, 9, 20 richtet sich nach dem gewünschten Synthesegas bzw. dem Produkt, welches in der Chemieanlage 11 oder der biotechnologischen Anlage aus dem Synthesegas hergestellt werden soll. Dabei erfolgt eine zusätzliche Anreicherung mit Wasserstoff, der in der Anlage 21 durch Wasserelektrolyse erzeugt wird.

## Patentansprüche

1. Anlagenverbund zur Stahlerzeugung mit
einem Hochofen (1) zur Roheisenerzeugung,
einem Konverterstahlwerk (2) zur Rohstahlerzeugung,
einem Gasleitungssystem für Gase, die bei der Roheisenerzeugung und/oder der Rohstahlerzeugung anfallen,
**dadurch gekennzeichnet, dass** der Anlagenverbund zusätzlich eine an das Gasleitungssystem angeschlossene Chemieanlage (11) oder Biotechnologieanlage sowie eine Anlage (21) zur Wasserstofferzeugung umfasst, wobei die Anlage (21) zur Wasserstofferzeugung durch eine wasserstoffführende Leitung (22) mit dem Gasleitungssystem verbunden ist, wobei der Anlagenverbund zusätzlich ein Kraftwerk (3) umfasst, welches als Gasturbinenkraftwerk oder Gasturbinen- und Dampfturbinenkraftwerk ausgelegt ist und mit einem Gas betrieben wird, welches zumindest eine Teilmenge des bei der Roheisenerzeugung im Hochofen (1) anfallenden Hochofengichtgases und/oder eine Teilmenge des in dem Konverterstahlwerk anfallenden Konvertergases (2) umfasst, und dass das Gasleitungssystem eine schaltbare Gasweiche (12) zur Aufteilung der dem Kraftwerk (3) und der Chemieanlage (11) oder Biotechnologieanlage zugeführte Gasmengenströme aufweist und wobei die Anlage (21) zur Wasserstofferzeugung eine Elektrolyseanlage zur Wasserelektrolyse aufweist.

2. Anlagenverbund nach Anspruch 1, **dadurch gekennzeichnet, dass** die wasserstoffführende Leitung (22) an eine in Strömungsrichtung vor der Chemie- oder Biotechnologieanlage (11) angeordnete Mischvorrichtung (13) angeschlossen ist, in der ein der Mischvorrichtung (13) zugeführter Gasstrom mit Wasserstoff angereichert wird.

3. Anlagenverbund nach Anspruch 1 oder 2, **dadurch gekennzeichnet, dass** die Chemieanlage (11) oder Biotechnologieanlage an eine Leitung für Konvertergas angeschlossen ist und dass die wasserstoffführende Leitung (22) mit der Konvertergasleitung verbunden ist, so dass das Konvertergas zur Verwendung in der Chemieanlage (11) oder Biotechnologieanlage mit Wasserstoff angereichert werden kann.

4. Anlagenverbund nach Anspruch 3, **dadurch gekennzeichnet, dass** die Elektrolyseanlage mittels einer Sauerstoffrückführeinrichtung mit dem Hochofen (1) und/oder einer Anlage zur Rohstahlerzeugung verbunden ist.

5. Verfahren zum Betreiben eines Anlagenverbundes, nach Anspruch 1 der einen Hochofen (1) zur Roheisenerzeugung, ein Konverterstahlwerk (2), eine Chemieanlage (12) oder Biotechnologieanlage sowie eine Anlage (21) zur Wasserstofferzeugung aufweist,
a) wobei zumindest eine Teilmenge eines bei der Roheisenerzeugung im Hochofen (1) anfallenden Hochofengichtgases und/oder eine Teilmenge eines bei der Rohstahlerzeugung anfallenden Konvertergases nach einer Gaskonditionierung als Nutzgas zur Herstellung chemischer Produkte (16) verwendet wird,
b) wobei das Nutzgas vor seiner Verwendung als Synthesegas mit Wasserstoff angereichert wird, der in der Anlage (21) zur Wasserstofferzeugung gebildet wird,
wobei ein Teil des Nutzgases einem Kraftwerk (3) zugeführt und zur Stromerzeugung verwendet wird, wobei das Kraftwerk (3) und die Chemie- oder Biotechnologieanlage (11) parallel geschaltet sind und wobei die einerseits dem Kraftwerk (3) und andererseits der Chemie- oder Biotechnologieanlage (11) zugeführten Gasteilströme geregelt werden und wobei der Wasserstoff durch Wasserelektrolyse erzeugt wird.

6. Verfahren nach Anspruch 5, **dadurch gekennzeichnet, dass** das Synthesegas aus Konvertergas erzeugt und mit Wasserstoff angereichert wird.

7. Verfahren nach Anspruch 5, **dadurch gekennzeichnet, dass** aus Hochofengichtgas und Konvertergas ein Mischgas hergestellt wird, welches nach einer Gaskonditionierung und Anreicherung mit Wasserstoff als Synthesegas zur Herstellung chemischer Produkte oder in der Biotechnologieanlage für biochemische Prozesse verwendet wird.

8. Verfahren nach einem der Ansprüche 5 bis 7, **dadurch gekennzeichnet, dass** die Wasserelektrolyse mit elektrischem Strom betrieben wird, der aus erneuerbarer Energie erzeugt wurde.

9. Verfahren nach Anspruch 7 oder 8, **dadurch gekennzeichnet, dass** der bei der Wasserelektrolyse gebildete Sauerstoff im Hochofen (1) zur Roheisenerzeugung und/oder im Konverterstahlwerk (2) zur Rohstahlerzeugung genutzt wird.

10. Verfahren nach einem der Ansprüche 5 bis 9, **dadurch gekennzeichnet, dass** die erzeugte H₂-Menge so bemessen wird, dass der Kohlenstoff- und Stickstoffanteil des Nutzgases vollständig zur Umwandlung in chemische Produkte genutzt werden kann.

11. Verfahren nach einem der Ansprüche 5 bis 10, **dadurch gekennzeichnet, dass** 5 % bis 60 % der Gasmenge, die bei der Roheisenerzeugung als Hochofengichtgas und/oder im Konverterstahlwerk (2) als Konvertergas anfällt, der Chemie- oder Biotechnologieanlage (11) zugeführt und zur Erzeugung chemischer Produkte (16) verwendet wird.

12. Verfahren nach einem der Ansprüche 5 bis 11, **dadurch gekennzeichnet, dass** dem Nutzgas Kokereigas zugemischt wird.

13. Verfahren nach einem der Ansprüche 5 bis 12, **dadurch gekennzeichnet, dass** das Kraftwerk (3) in einen Teillast- oder Grundlastbetrieb heruntergefahren wird sowie der dem Kraftwerk (3) zugeführte Gasmengenstrom entsprechend gedrosselt wird, wenn der Strompreis für elektrische Energie aus erneuerbarer Energie um einen vorgegebenen Faktor kleiner ist als die Kosten des im Kraftwerk (3) erzeugten Stromes.

14. Verwendung einer Chemie- oder Biotechnologieanlage (11) im Verbund mit einer Anlage (21) zur Wasserstofferzeugung zur Ankopplung an einem Anlagenverbund gemäß Anspruch 1, welches zumindest einen Hochofen (1) zur Roheisenerzeugung und ein Konverterstahlwerk (2) umfasst, mit der Maßgabe, dass zumindest eine Teilmenge des bei der Roheisenerzeugung anfallenden Hochofengichtgases und/oder zumindest eine Teilmenge des im Konverterstahlwerk anfallenden Konvertergases der Chemie- oder Biotechnologieanlage (11) zugeführt und nach einer Gaskonditionierung und nach einer Anreicherung mit Wasserstoff als Synthesegas zur Erzeugung chemischer Produkte genutzt wird.

## Claims

1. Plant complex for steel production comprising
a blast furnace (1) for producing pig iron,
a converter steel works (2) for producing crude steel,
a gas-conducting system for gases that occur in the production of pig iron and/or
the production of crude steel,
**characterized in that** the plant complex additionally comprises a chemical plant (11) or biotechnological plant connected to the gas-conducting system and a plant (21) for producing hydrogen, wherein the plant (21) for producing hydrogen is connected to the gas-conducting system by a hydrogen-carrying line (22), wherein the plant complex additionally comprises a power-generating plant (3) which is designed as a gas-turbine power-generating plant or gas-turbine and steam-turbine power-generating plant and is operated with a gas that comprises at least a partial amount of the blast-furnace top gas that occurs in the production of pig iron in the blast furnace (1) and/or a partial amount of the converter gas (2) that occurs in the converter steel works, and **in that** the gas-conducting system has a switchable gas diverter (12) for dividing the streams of gas that are fed to the power-generating plant (3) and the chemical plant (11) or biotechnological plant and wherein the plant (21) for producing hydrogen has an electrolysis plant for the electrolysis of water.

2. Plant complex according to Claim 1, **characterized in that** the hydrogen-carrying line (22) is connected to a mixing device (13), which is arranged upstream of the chemical or biotechnological plant (11), as seen in the flow direction, and in which a stream of gas which is fed to the mixing device (13) is enriched with hydrogen.

3. Plant complex according to Claim 1 or 2, **characterized in that** the chemical plant (11) or biotechnological plant is connected to a line for converter gas, and **in that** the hydrogen-carrying line (22) is connected to the converter-gas line, so that the converter gas, for use in the chemical plant (11) or biotechnological plant, can be enriched with hydrogen.

4. Plant complex according to Claim 3, **characterized in that** the electrolysis plant is connected to the blast furnace (1) and/or to a plant for producing crude steel by means of an oxygen-return device.

5. Method for operating a plant complex according to Claim 1 which has a blast furnace (1) for producing pig iron, a converter steel works (2), a chemical plant (12) or biotechnological plant and a plant (21) for producing hydrogen,
a) at least a partial amount of a blast-furnace top gas that occurs in the production of pig iron in the blast furnace (1) and/or a partial amount of a converter gas that occurs in the production of crude steel being used, after a gas-conditioning operation, as a useful gas for producing chemical products (16),
b) wherein the useful gas, prior to being used as syngas, is enriched with hydrogen that is formed in the plant (21) for producing hydrogen,
wherein part of the useful gas is fed to a power-generating plant (3) and used for producing power, wherein the power-generating plant (3) and the chemical or biotechnological plant (11) are connected in parallel, and wherein the partial streams of gas that are fed to the power-generating plant (3), on the one hand, and the chemical or biotechnological plant (11), on the other hand, are controlled and wherein the hydrogen is produced by electrolysis of water.

6. Method according to Claim 5, **characterized in that** the syngas is produced from converter gas and is enriched with hydrogen.

7. Method according to Claim 5, **characterized in that** blast-furnace top gas and converter gas are used to produce a mixed gas which, after a gas-conditioning operation and enrichment with hydrogen, is used as syngas for producing chemical products or in the biotechnological plant for biochemical processes.

8. Method according to one of Claims 5 to 7, **characterized in that** the electrolysis of water is operated by electric power which has been produced from renewable energy.

9. Method according to Claim 7 or 8, **characterized in that** the oxygen formed during the electrolysis of water is used within the blast furnace (1) for producing pig iron and/or in the converter steel works (2) for producing crude steel.

10. Method according to one of Claims 5 to 9, **characterized in that** the amount of H₂ produced is set such that the entire fraction of carbon and nitrogen in the useful gas can be used for converting into chemical products.

11. Method according to one of Claims 5 to 10, **characterized in that** 5% to 60% of the amount of gas that occurs as blast-furnace top gas during the production of pig iron and/or as converter gas in the converter steel works (2) is fed to the chemical or biotechnological plant (11) and used for producing chemical products (16).

12. Method according to one of Claims 5 to 11, **characterized in that** coke-oven gas is admixed with the useful gas.

13. Method according to one of Claims 5 to 12, **characterized in that** the power-generating plant (3) is shut down to a partialload or a base-load operation, and the stream of gas fed to the power-generating plant (3) is restricted correspondingly, if the power price for electrical energy obtained from renewable energy is a predetermined factor lower than the costs of the power produced in the power-generating plant (3).

14. Use of a chemical or biotechnological plant (11) in conjunction with a plant (21) for producing hydrogen, for coupling to a plant complex according to Claim 1 that comprises at least a blast furnace (1) for producing pig iron and a converter steel works (2), with the proviso that at least a partial amount of the blast-furnace top gas that occurs in the production of pig iron and/or at least a partial amount of the converter gas that occurs in the converter steel works are fed to the chemical or biotechnological plant (11) and, after a gas-conditioning operation and after an enrichment with hydrogen, are used as syngas for producing chemical products.

## Revendications

1. Ensemble d'installations pour la production d'acier, comprenant un haut-fourneau (1) pour la production de fonte brute, une aciérie à convertisseur (2) pour la production d'acier brut,
un réseau de conduites de gaz pour les gaz émanant de la production de fonte brute et/ou de la production d'acier brut,
**caractérisé en ce que**
l'ensemble d'installations comprend additionnellement une installation chimique (11) ou une installation biotechnologique raccordée sur le réseau de conduites de gaz, ainsi qu'une installation (21) de production d'hydrogène, l'installation (21) de production d'hydrogène étant reliée par une conduite (22) de passage d'hydrogène avec le réseau de conduites de gaz, l'ensemble d'installations comprenant additionnellement une centrale électrique (3), laquelle est conçue sous la forme d'une centrale électrique à turbine à gaz ou d'une centrale électrique à turbine à gaz et turbine à vapeur et est exploitée avec un gaz, lequel comprend au moins une quantité partielle du gaz de haut-fourneau émanant de la production de fonte brute dans le haut-fourneau (1) et/ou une quantité partielle du gaz de convertisseur (2) émanant de l'aciérie à convertisseur, et **en ce que** le réseau de conduites de gaz comporte un embranchement de gaz (12) commutable, destiné à répartir les flux gazeux amenés vers la centrale électrique (3) et vers l'installation chimique (11) ou l'installation biotechnologique et **en ce que** l'installation (21) de production d'hydrogène comporte une installation d'électrolyse, pour l'électrolyse de l'eau.

2. Ensemble d'installations selon la revendication 1, **caractérisé en ce que** la conduite (22) de passage d'hydrogène est raccordée sur un dispositif mélangeur (13) placé en amont de l'installation chimique ou biotechnologique (11) dans la direction d'écoulement, dans lequel un flux gazeux amené vers le dispositif mélangeur (13) est enrichi en hydrogène.

3. Ensemble d'installations selon la revendication 1 ou 2, **caractérisé en ce que** l'installation chimique (11) ou l'installation biotechnologique est raccordée sur une conduite de gaz de convertisseur et **en ce que** la conduite (22) de passage d'hydrogène est reliée avec la conduite de gaz de convertisseur, de telle sorte que le gaz de convertisseur puisse être enrichi en hydrogène, pour être utilisé dans l'installation chimique (11) ou dans l'installation biotechnologique.

4. Ensemble d'installations selon la revendication 3, **caractérisé en ce que** l'installation d'électrolyse est reliée au moyen d'un système de recyclage d'oxygène avec le haut-fourneau (1) et/ou avec une installation de production d'acier brut.

5. Procédé destiné à faire fonctionner un ensemble d'installations selon la revendication 1, qui comporte un haut-fourneau (1) pour la production de fonte brute, une aciérie à convertisseur (2), une installation chimique (12) ou biotechnologique, ainsi qu'une installation (21) de production d'hydrogène,
a) après un conditionnement de gaz, au moins une quantité partielle du gaz de haut-fourneau émanant de la production de fonte brute dans le haut-fourneau (1) et/ou une quantité partielle du gaz de convertisseur émanant de l'aciérie à convertisseur étant utilisée comme gaz utile, pour la fabrication de produits chimiques (16),
b) avant son utilisation comme gaz synthétique, le gaz utile étant enrichi en hydrogène, qui est créé dans l'installation (21) de production d'hydrogène,
une partie du gaz utile étant amenée vers une centrale électrique (3) et utilisée pour produire de l'électricité, la centrale électrique (3) et l'installation chimique ou biotechnologique (11) étant montées en parallèle et les flux amenés d'une part vers la centrale électrique (3) et d'autre part vers l'installation chimique ou biotechnologique (11) étant régulés et en ce que l'hydrogène est produit par électrolyse de l'eau.

6. Procédé selon la revendication 5, **caractérisé en ce que** le gaz synthétique est produit à partir de gaz de convertisseur et enrichi en hydrogène.

7. Procédé selon la revendication 5, **caractérisé en ce qu'**un gaz mixte est fabriqué à partir du gaz de haut-fourneau et du gaz de convertisseur, qui, après un conditionnement de gaz et un enrichissement en hydrogène, est utilisé comme gaz synthétique pour la fabrication de produits chimiques ou dans l'installation biotechnologique, pour des processus biochimiques.

8. Procédé selon l'une quelconque des revendications 5 à 7, **caractérisé en ce que** l'électrolyse de l'eau fonctionne avec du courant électrique qui a été produit à partir d'une énergie renouvelable.

9. Procédé selon la revendication 7 ou 8, **caractérisé en ce que** l'oxygène créé lors de l'électrolyse de l'eau est utilisé dans le haut-fourneau (1) pour la production de fonte brute et/ou dans l'aciérie à convertisseur (2) pour la production d'acier brut.

10. Procédé selon l'une quelconque des revendications 5 à 9, **caractérisé en ce que** la quantité produite de H₂ est calculée de telle sorte que la part de carbone ou d'azote dans le gaz utile puisse être utilisée totalement pour la transformation en produits chimiques.

11. Procédé selon l'une quelconque des revendications 5 à 10, **caractérisé en ce que** de 5 % à 60 % de la quantité de gaz émanant de la production de fonte brute comme gaz de haut-fourneau et/ou comme gaz de convertisseur dans l'aciérie à convertisseur (2) sont amenés vers l'installation chimique ou biotechnologique (11) et utilisés pour produire des produits chimiques (16).

12. Procédé selon l'une quelconque des revendications 5 à 11, **caractérisé en ce qu'** on ajoute par mélange au gaz utile du gaz de cokerie.

13. Procédé selon l'une quelconque des revendications 5 à 12, **caractérisé en ce qu'** on fait baisser la centrale électrique (3) dans un mode en charge partielle ou en charge de base, et on étrangle en conséquence le flux gazeux amené vers la centrale électrique (3) lorsque le prix du courant pour l'énergie électrique généré à partir d'énergie renouvelable est inférieur d'un coefficient prédéfini aux coûts du courant produit dans la centrale électrique (3).

14. Utilisation d'une installation chimique ou biotechnologique (11) en association avec une installation (21) de production d'hydrogène pour l'accouplement sur un ensemble d'installations selon la revendication 1, laquelle comprend au moins un haut-fourneau (1) pour la production de fonte brute et une aciérie à convertisseur (2), avec le critère qu'au moins une quantité partielle du gaz de haut-fourneau émanant de la production de fonte brute et/ou au moins une quantité partielle du gaz de convertisseur émanant de l'aciérie à convertisseur soit amenée vers l'installation chimique ou biotechnologique (11) et après un conditionnement de gaz et après un enrichissement avec de l'hydrogène, soit utilisée comme gaz de synthèse pour la production de produits chimiques.
